# EUROPEAN PATENT APPLICATION

(11) **EP 4 687 148 A1**
(43) Date of publication of application: **04.02.2026**
(21) Application number: 25192857.8
(22) Date of filing: 30.07.2025
(51) Int. Cl.: G16H 20/30

(54) **SYSTEM FOR MANAGING THE AUTHORIZATION TO PROCEED WITH AN INTAKE OF A DRUG DOSE BY A USER AS A FUNCTION OF THE PHYSICAL ACTIVITY PERFORMED BY THE USER AND RELATED METHOD**

(30) Priority: 02.08.2024 IT 202400018229
(71) Applicant: Technogym S.p.A., 47521 Cesena (FC) (IT)
(72) Inventor: ZANUSO, Silvano, I-47521 Cesena (FORLI'-CESENA) (IT); ZOFFOLI, Luca, I-47521 Cesena (FORLI'-CESENA) (IT); PASINI, Alessandro, I-47521 Cesena (FORLI'-CESENA) (IT)
(74) Representative: Mozzi, Matteo

(57) **Abstract**

System (100) for managing the authorisation to proceed with the intake by a user of a dose as a function of the physical activity performed by the user, said system (100) comprising: a first electronic assembly (1) for tracking the physical activity performed by the user; at least one first data processing unit (2) operatively connected to the first electronic assembly (1) for tracking the physical activity performed by the user; at least one first memory unit (3) operatively connected to the at least one first data processing unit (2). The at least one first data processing unit (2) is configured to: compare a value (Q1) of the amount of physical exercise performed by the user, starting from a first instant in time in which a drug dose was taken by the user, with a set reference value (R1), the value (Q1) of the amount of physical exercise performed by the user having been determined, starting from the first instant in time in which a drug dose was taken by the user, as a function of data representative of the physical activity performed by the user traced by said first electronic assembly (1) for tracking the physical activity performed by the user; store in the at least one first memory unit (3) the determined value (Q1) of the amount of physical exercise performed by the user. If the determined value (Q1) of the amount of physical exercise is greater than or equal to the set reference value (R1), the at least one first data processing unit (2) is configured to generate first information (AP-1) representative of an authorisation for the user to proceed with the intake of the next drug dose and to provide the user and/or to personnel qualified to authorise the purchase of the drug with the generated first information (AP-1) representative of the authorisation for the user to proceed with the intake of the next drug dose. If the determined value (Q1) of the amount of physical exercise is lower than the set reference value (R1), the at least one first data processing unit (2) is configured to generate and provide to the user and/or to personnel qualified to authorise the purchase of the drug second information (AP-2) representative of a non-authorisation for the user to proceed with the intake of the next drug dose or not to generate any first information (AP-1) representative of an authorisation for the user to proceed with the intake of the next drug dose.

## Description

### Field of the invention

The present invention relates to the health and fitness sector and, in particular, to a system for managing the authorization to proceed with an intake of a drug dose by a user as a function of the physical activity performed by the user and to a related method.

### Technological background of the invention

A class of drugs (whose application and effectiveness has been recently discovered) is known, which is capable of treating and curing specific pathologies (such as obesity and type 2 diabetes) substantially by mimicking the effects of a substance called GLP-1 (glucagon-like peptide-1 receptor) present in the human body.

GLP-1 is in fact a hormone that prepares the body to use the nutrients ingested through the meal and, in particular, on one hand, causes an increase in insulin preparing the body to regulate blood glucose levels and, on the other hand, slows down gastric emptying thereby increasing the sensation of satiety.

In addition to the treatment with such a drug, which may furthermore be subject to authorization for purchase (for example through a prescription by personnel qualified to issue an authorization for the purchase of the drug), the performance of physical activity also plays an important role in the treatment of such pathologies, in particular the performance of strength exercises or, even, combined cardiovascular and strength exercises.

In the fight against obesity, the integration between a new type of drug, the glucagon-like peptide-1 (GLP-1) receptor agonists, and physical exercise represents an effective combination if the goal is sustainable weight loss and improving overall health.

These drugs act on specific hormones in the body that contribute to regulating insulin, controlling blood sugar levels, and managing appetite. They therefore not only improve overall metabolic health and glycemic control, but also promote weight loss processes.

As with many treatments aimed at weight loss, the reduction of fat mass promoted by GLP-1 agonists may inadvertently cause considerable reductions also of lean mass, including at the muscular tissue level.

Those who take these drugs therefore undergo both a loss of fat mass and muscular mass.

In consideration of this potential challenge, the combination of GLP-1 agonists with structured training appears as a promising strategy to achieve proper weight management.

While GLP-1 agonists on the other hand effectively suppress appetite and promote weight reduction, on the other hand the inclusion of strength training in training routines balances the loss of muscular mass, further enhancing the positive results of the treatment.

Therefore, on the one hand, the performance of strength exercises or combined cardiovascular and strength exercises can make the action of the drug itself more effective, thereby enhancing it, and, on the other hand, it may not enhance the effect of the drug but contribute to reducing possible side effects of the drug itself, for example, the loss of muscular mass, by preventing/contrasting the consumption of muscular mass caused by weight loss, thereby preserving lean mass.

Therefore, the best possible treatment of obesity and type 2 diabetes and the achievement of the benefit for the user are obtainable by taking the drug corresponding to the GLP-1 hormone and performing physical activity, preferably strength exercises.

Indeed, the so-called "strength training" (also known as strength training or counter resistance training) involves exercises that work on the muscles with resistance activities (such as weight lifting or the use of resistance bands) and offers multiple benefits that go beyond normal aerobic exercise, including increased muscular mass, enhancement of metabolism, improvement in strength and functional capacity.

Strength training represents a powerful remedy against muscular mass loss, stimulating the growth and maintenance of the musculature.

If practiced regularly, it allows patients to mitigate the muscular loss associated with GLP-1 agonists, ensuring that weight loss mainly affects fat mass deposits rather than lean muscular mass.

The integration of strength training into the treatment regimen not only safeguards muscular mass, but also improves metabolic health. Muscles are metabolically active tissues that contribute to energy expenditure and the regulation of glucose levels. The increase in muscular mass through strength training accelerates metabolism, strengthens insulin sensitivity, and promotes a better glycemic control, in synergy with the benefits brought by GLP-1 agonists at the metabolic level.

Thanks to the synergy between drug therapy and targeted physical exercise, patients can achieve sustainable weight loss, preserve muscular mass and improve their metabolic health and functional abilities.

The emphasis on the importance of strength training in combination with GLP-1 therapy highlights the value of personalized physical activity regimens in maximizing therapeutic success and improving the well-being of those who struggle with obesity.

However, while physical activity alone certainly does not effectively help to counter such pathologies and a drug dose alone might not be sufficient to obtain the desired benefit, a combination between drug dose taken and physical activity performed, if not adequately controlled, could even risk making the drug treatment less effective in the case in which, for example, a side effect occurs or the effect of the drug ends but the user continues persistently with physical activity.

In consideration of the above, it is clear that such treatment can be further improved to bring optimal benefit to the user by controlling the drug dose taken by the user as a function of the activity performed by the user starting, for example, from the moment in which the drug dose is taken or, in general, as a function of the previously taken drug dose, precisely to be able, on the one hand, to establish the correct moment to take a subsequent drug dose and, on the other hand, to monitor the procurement of the drug itself.

In this scenario, there is a strong need to have a system for managing the authorization to proceed with an intake of a drug dose by a user as a function of the physical activity performed by the user, which allows to optimize the benefit effect for the user, also avoiding side effects due to the drug, by controlling the drug dose taken by the user precisely as a function of the activity performed by the user starting, for example, from the moment in which the drug dose is taken and, on the one hand, to establish the most appropriate moment to take the drug dose again and, on the other hand, to monitor the procurement of the drug in order to possibly establish the need to obtain a new authorization for purchase.

### Summary

The object of the present invention is to devise and provide a system for managing the authorization to proceed with an intake of a drug dose by a user as a function of the physical activity performed by the user, which allows the user to achieve the benefit effect, also avoiding side effects due to the drug, by controlling the drug dose taken by the user precisely as a function of an amount of activity performed by the user starting, for example, from the moment in which the drug dose is taken and, on the one hand, to establish the most appropriate moment to take the drug dose again and, on the other hand, to monitor the procurement of the drug in order to possibly establish the need to obtain a new authorization for purchase.

Said object is achieved by a system according to claim 1.

The invention also relates to a method for managing the authorization to proceed with an intake of a drug dose by a user as a function of the physical activity performed by the user.

Preferred embodiments of the system and method are defined in the respective dependent claims.

### Brief description of the figures

Further features and advantages of the system and of said method according to the invention will be clear from the following description of preferred embodiments, given by way of example and not limitation, with reference to the accompanying drawings, wherein:
- Figures 1a-1c illustrate examples of exercise machines employable in the system for managing the authorization to proceed with an intake of a drug dose by a user as a function of the physical activity performed by the user according to the present invention;
- Figure 2 illustrates, by means of a block diagram, a system for managing the authorization to proceed with an intake of a drug dose by a user as a function of the physical activity performed by the user according to the present invention;
- Figure 3 illustrates, by means of a block diagram, a system for managing the authorization to proceed with an intake of a drug dose by a user as a function of the physical activity performed by the user according to one embodiment;
- Figure 4 illustrates, by means of a block diagram, a system for managing the authorization to proceed with an intake of a drug dose by a user as a function of the physical activity performed by the user according to a further embodiment;
- Figure 5 illustrates, by means of a block diagram, a system for managing the authorization to proceed with an intake of a drug dose by a user as a function of the physical activity performed by the user according to a further embodiment; and
- Figure 6 schematically illustrates, by means of a block diagram, a method for managing the authorization to proceed with an intake of a drug dose by a user as a function of the physical activity performed by the user, according to the present invention.

It is noted that in the above figures identical or similar elements are denoted by the same numeric and/or alphanumeric reference.

### Detailed description

With reference to the above figures, a system 100 for managing the authorization to proceed with an intake of a drug dose by a user as a function of the physical activity performed by the user is now described, hereinafterr also referred to as management system or simply system, according to the present invention.

For the purposes of the present invention, "drug" means any "exercise-sensitive" drug (or compound), that is, a drug whose effect, for the user, could be enhanced by performing physical exercise and/or whose side effect, for the user, could be reduced always by performing physical exercise.

An example of such a drug may be a drug for diabetes (type 2) and for obesity, such as a drug that mimics the effects of the GLP-1 (glucagon-like peptide-1 receptor) substance present in the human body.

Such a drug may be subject, among other things, to authorization for purchase (for example through a prescription by personnel qualified to issue the prescription).

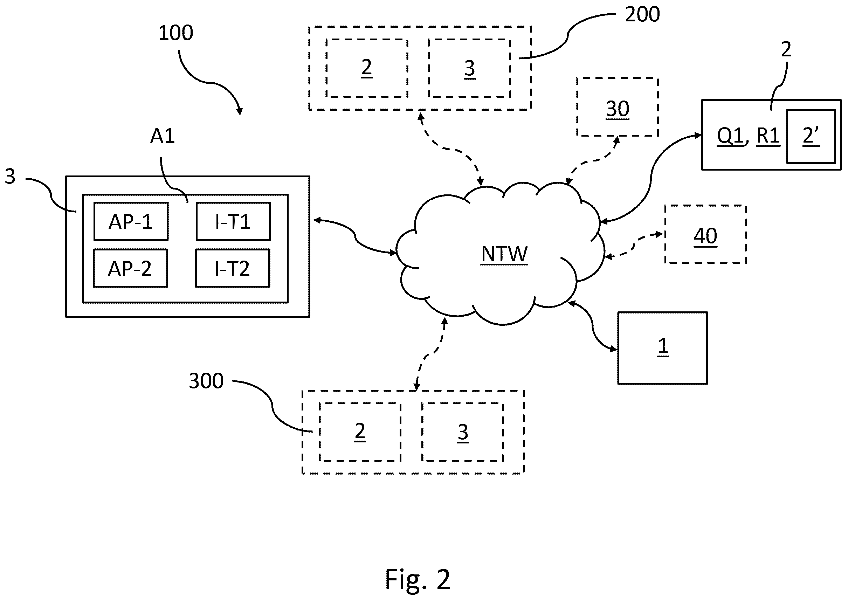

According to the present invention, with reference to figure 2, the system 100 comprises a first electronic assembly 1 for tracking the physical activity performed by the user, hereinafter also referred to as first electronic tracking assembly 1 or simply first electronic assembly 1.

By "electronic assembly for tracking the physical activity of the user" it is meant the set of at least one sensor module and at least one data processing module operatively connected to one another to communicate data.

Examples of the first electronic assembly for tracking the physical activity will be described below with reference to different embodiments of the present invention.

The system 100 further comprises at least one first data processing unit 2 operatively connected to the first electronic assembly 1 for tracking the physical activity performed by the user.

The at least one first data processing unit 2 is operatively connected to the first electronic assembly 1, in a wired or wireless mode, for example via a data communication network NTW (for example, Internet), schematically shown in figure 2.

The at least one first data processing unit 2 is, for example, a microcontroller or a microprocessor.

The system 100 further comprises at least one first memory module 2' operatively connected to the at least one first data processing unit 2.

The at least one first memory module 2' may be internal (as schematically illustrated in figure 2) or external to the at least one first data processing unit 2 (embodiment not shown in the figures).

The at least one first data processing unit 2, by loading and executing one or more program codes stored in the at least one first memory module 2', is configured to control the system 100 and to perform operational steps of the related method, as will be described below.

According to the present invention, the system 100 further comprises at least one first memory unit 3 operatively connected to the at least one first data processing unit 2.

In greater detail, the at least one first memory unit 3 is operatively connected to the at least one first data processing unit 2, in a wired or wireless mode, for example via the data communication network NTW (for example, Internet), schematically shown in figure 2.

According to the present invention, from a functional point of view, the at least one first data processing unit 2 is configured to perform the following operations.

The at least one first data processing unit 2 is configured to compare a value Q1 of the amount of physical exercise performed by the user, starting from a first time instant in which a drug dose is taken by the user, with a set reference value R1.

The value Q1 of the amount of physical exercise performed by the user has been determined, starting from the first time instant in which a drug dose is taken by the user, as a function of data representative of the physical activity performed by the user tracked by said first electronic assembly 1 for tracking the physical activity performed by the user.

The value Q1 of the amount of physical exercise performed by the user is therefore an increasing value over time, starting from the first time instant in which a drug dose is taken by the user, with the performance by the user of physical exercise in subsequent training sessions.

Returning to the invention, the at least one first data processing unit 2 is configured to store in the at least one first memory unit 3 the determined value Q1 of the amount of physical exercise performed by the user.

Therefore, the determined value Q1 of the amount of physical exercise performed by the user is stored each time it is determined, thus the at least one first memory unit 3 stores the last determined value, that is, the most updated one.

Still with reference to the invention, the at least one first data processing unit 2 is configured to, (Y) if the determined value Q1 of the amount of physical exercise is greater than or equal to the set reference value R1:
- generate a first information AP-1 representative of an authorization for the user to take the next drug dose;
- provide the user and/or personnel qualified to issue an authorization for the purchase of the drug with the generated first information AP-1 representative of the authorization for the user to take the next drug dose.

Therefore, the at least one first data processing unit 2, in the event that the determined value Q1 representative of the amount of physical exercise performed by the user is greater than or equal to the set reference value R1, generates the first information AP-1 representative of an authorization for the user to take the next drug dose as it is necessary for the user to take a next drug dose, considering that the result of the comparison indicates that the beneficial effect of the previous drug dose has worn off and/or there is still a risk of a side effect of the drug during the performance of physical exercise.

The at least one first data processing unit 2 is configured to, (N) if the determined value Q1 of the amount of physical exercise is lower than the set reference value R1:
- generate and provide the user and/or personnel qualified to issue an authorization for the purchase of the drug with a second information AP-2 representative of a non-authorization for the user to take the next drug dose or generate no first information AP-1 representative of an authorization for the user to take the next drug dose.

In other words, the at least one first data processing unit 2, in the event that the determined value Q1 representative of the amount of physical exercise performed by the user is lower than the set reference value R1, generates a second information AP-2 representative of a non-authorization for the user to take the next drug dose or generates no first information AP-1 representative of an authorization for the user to take the next drug dose as the user has not satisfied the necessary condition to obtain the authorization to take a next drug dose or that the drug dose will need to be changed.

According to one embodiment, the at least one first data processing unit 2 is configured to determine the value Q1 of the amount of physical exercise performed by the user.

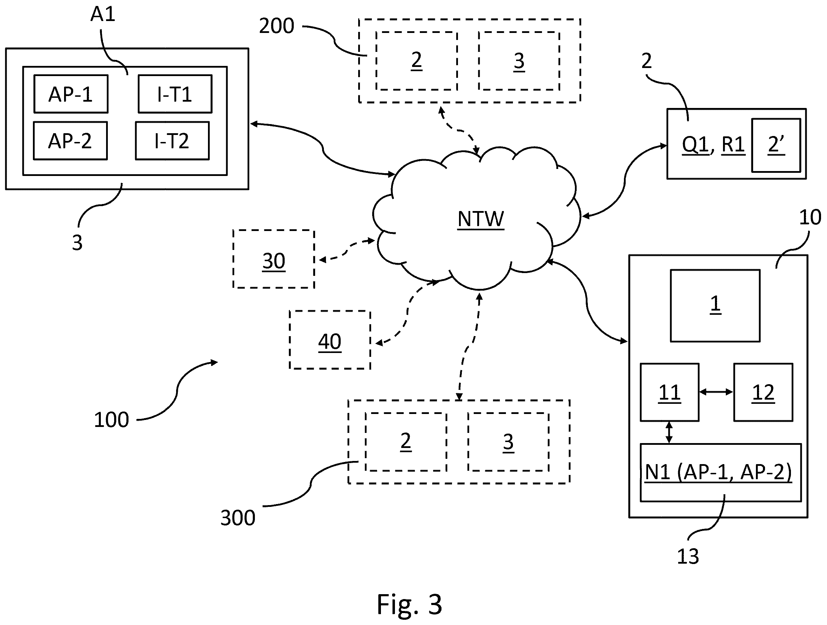

According to one embodiment, in combination with any of the preceding embodiments and in figure 3, the system 100 comprises an exercise machine 10 operatively connected to the at least one first data processing unit 2.

In greater detail, the exercise machine 10 is operatively connected to the at least one first data processing unit 2, in a wired or wireless mode, for example via the data communication network NTW (for example, Internet), schematically shown in figure 3.

By "exercise machine" it is meant any exercise machine employable by a user for performing strength exercises, for performing cardiovascular exercises or for performing combined cardiovascular and strength exercises.

Therefore, examples of exercise machines may be strength training machines, in which the user during the execution of an exercise moves a training or workload thereby strengthening one or more parts of the body (chest, shoulders, upper limbs, lower limbs and so on) or so-called cardiovascular training machines such as, for example, treadmills, bikes, rowers and so on.

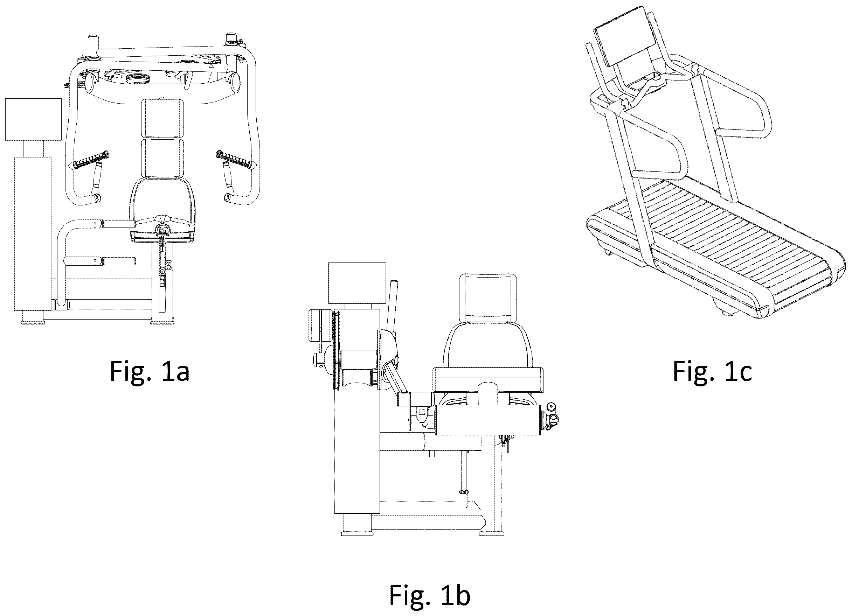

In the example of figures 1a-1c, respectively, a strength training machine for the upper limbs (figure 1a - "chest press"), a strength training machine for the lower limbs (figure 1b - "leg extension") and a cardiovascular training machine (figure 1c - "treadmill") are shown.

In this embodiment, the exercise machine 10 comprises said first electronic assembly 1 for tracking the physical activity performed by the user.

In this regard, the first electronic assembly 1 for tracking the physical activity performed by the user comprises, in this case, one or more sensor modules adapted to perform measurements of one or more operating parameters of the exercise machine 10 and at least one data processing module adapted to determine the aforesaid data representative of the physical activity performed by the user (for example, movement data of the user on the exercise machine 10) based on the measurements performed by said one or more sensor modules.

For example, the at least one data processing module of the first electronic assembly 1 is configured to determine the following operating parameters of the exercise machine by means of the measurements provided by the one or more sensor modules:
- a belt speed value of a treadmill or a rotational speed value of at least one of the rollers that drive the treadmill belt in rotation, measured for example by an encoder;
- an inclination value (slope) of the treadmill belt, measured for example by the actuator that adjusts the inclination of the belt using a position or other type of sensor;
- a rotational speed value of the cranks of a stationary bicycle, measured for example by an encoder;
- a pedalling resistance value obtained by means of an electromagnetic brake, measured for example by a current sensor detecting the electric current flowing through the electromagnetic brake, the intensity value of the electric current flowing through the electromagnetic brake being correlated to the braking (pedalling resistance) exerted by the electromagnetic brake;
- a lifted load value during the use of a strength training machine with gravitational load by weight stack with automatic detection of the lifted load by means of a sensor comprising, for example, load cells to determine the portion of weights of the weight stack lifted by the user, or for a strength training machine with an electronic resistance unit, for example of the electric motor type, by means of a current sensor detecting the electric current flowing through the electric motor, the intensity value of the electric current flowing through the electric motor being correlated to the resistance exerted by the electric motor to the movement of the levers or cable connected to the electric motor and moved by the user during the performance of the physical exercise;
- a range of motion value during the use of a strength training machine with gravitational load by weight stack by means of an optical sensor adapted to measure the range of motion value of the weight stack, or for a strength training machine with a generic resistance unit by means of a sensor that measures a range of motion value of the levers or of the cable connected to the resistance and moved by the user during the performance of the physical exercise.

Returning to the embodiment of figure 3 in which the system 100 comprises the exercise machine 10, the physical exercise for which the amount value Q1 is determined, is at least partially performed by the user using the exercise machine 10.

According to one embodiment, in combination with the previous and shown in figure 3, the exercise machine 10 comprises a respective first data processing block 11 and a respective first data communication block 12 operatively connected to the first data processing block 11.

The first data processing block 11 of the exercise machine 10 is configured to communicate data by means of said first data communication block 12.

In one embodiment, in combination with the previous one, the first data processing block 11 of the exercise machine 10 comprises the at least one first data processing unit 2.

In other words, the operations described above and also those subsequently provided for in the operation of the at least one first data processing unit 2 in accordance with different embodiments, are delegated to the exercise machine 10, in particular to the first data processing block 11.

According to one embodiment, as an alternative to the previous one, the first data processing block 11 of the exercise machine 10 is distinct from the at least one first data processing unit 2.

In this embodiment, the first data processing block 11 of the exercise machine 10 is operatively connected to the at least one first data processing unit 2.

In this embodiment, the first data processing block 11 of the exercise machine 10 is configured to communicate data to the at least one first data processing unit 2 by means of said first data communication block 12.

In this embodiment, from a functional point of view, the first data processing block 11 of the exercise machine 10 is configured to determine the value Q1 of the amount of physical exercise performed by the user, as defined above.

Furthermore, the first data processing block 11 of the exercise machine 10 is configured to transmit to the at least one first data processing unit 2, by means of said first data communication block 12, the determined value Q1 of the amount of physical exercise performed by the user.

Therefore, in this embodiment, the operation of determining the value Q1 of the amount of physical exercise performed by the user is delegated to the first data processing block 11 of the exercise machine 10 (and not to the at least one first data processing unit 2) whereas all the other operations described above and also those subsequently provided for in the operation of the at least one first data processing unit 2 in accordance with different embodiments remain effectively the responsibility of the at least one first data processing unit 2.

According to one embodiment, in combination with any of the preceding ones wherein the exercise machine 10 and the first data processing block 11 are provided and shown in figure 3, the exercise machine 10 comprises a respective first user interface module 13 operatively connected to the first data processing block 11 of the exercise machine 10.

In this embodiment, the at least one first data processing unit 2 is configured to send, based on the comparison between the determined value Q1 of the amount of physical exercise and the set reference value R1, a first notification message N1 comprising the first information AP-1 or the second information AP-2.

In this embodiment, the first data processing block 11 is configured to provide the user with the first notification message N1 by means of the first user interface module 13 of the exercise machine 10.

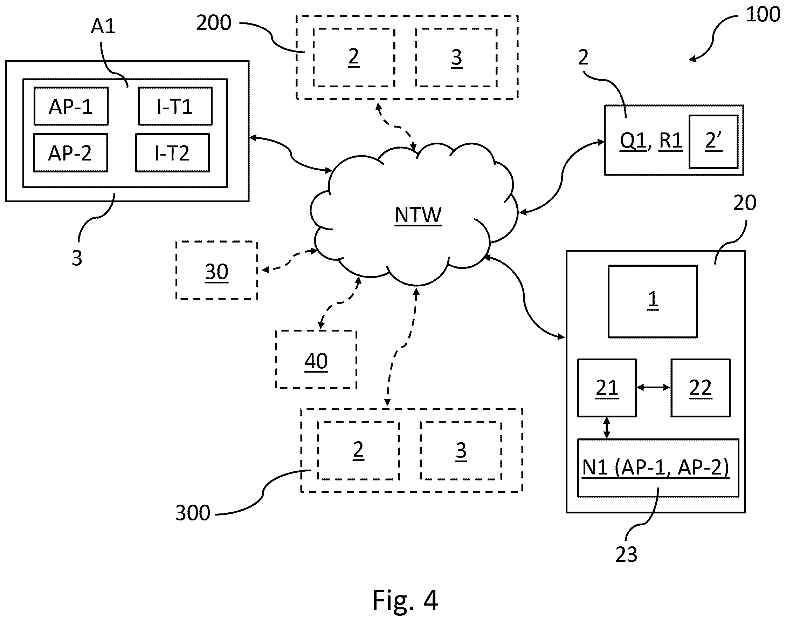

According to one embodiment, in combination with any of the preceding ones in which the exercise machine 10 is not provided and shown in figure 4, the system 100 comprises at least one user's portable electronic device 20 operatively connected to the at least one data processing unit 2.

The at least one user's portable electronic device 20 comprises said first electronic assembly 1 for tracking the physical activity performed by the user.

The first electronic assembly 1 for tracking the physical activity performed by the user employable in this case will be described below.

In greater detail, in this embodiment, the at least one user's portable electronic device 20 may be a device wearable by the user such as, for example, a bracelet or a smartwatch, or a portable device associable with the user such as, for example, a smartphone that may be associated with a part of the user's body (for example, smartphone housed in an armband of the user).

In this embodiment, the physical exercise for which the amount value Q1 is determined is therefore any exercise that can be performed by the user equipped with the at least one user's portable electronic device 20, worn or associated with the user, for example, running, walking, cycling, squats, jumps or any other bodyweight exercise.

In this regard, the first electronic assembly 1 for tracking the physical activity performed comprises, in this case, one or more sensor modules adapted to perform measurements of one or more parameters relating to the at least one user's portable electronic device 20, associable with the movement of the user, and at least one data processing module adapted to determine the aforesaid data representative of the physical activity performed by the user (for example, movement data of the user) on the basis of the measurements performed by said one or more sensor modules.

For example, the at least one data processing module of the first electronic assembly 1 is configured to determine the following parameters relating to the at least one user's portable electronic device 20 by means of the measurements provided by the one or more sensor modules:
- the distance covered by the user of the first electronic assembly 1, thus by the user, during the performance of the physical activity, by means of the position (and possibly its variation over time) detected for example by a GPS module;
- the time taken by the user to cover such distance;
- the number of repetitions of a bodyweight exercise (for example: squats, jumps, and so on) during the performance of the physical activity, by means of the measurement of the acceleration to which the first electronic assembly 1 is subjected as a result of the user's movement, for example through the measurements provided by an accelerometer;
- the speed of the user along a route performed, by means of the measurement of the acceleration to which the first electronic assembly 1 is subjected as a result of the user's movement, for example through the measurements provided by an accelerometer.

Returning to the embodiment of figure 4 in which the system 100 comprises the at least one user's portable electronic device 20, the physical exercise for which the amount value Q1 is determined is at least partially performed by the user using the at least one user's portable electronic device 20.

According to one embodiment, in combination with the previous and shown in figure 4, the at least one user's portable electronic device 20 comprises a respective first data processing block 21 and a respective first data communication block 22 operatively connected to the first data processing block 21.

The first data processing block 21 of the at least one user's portable electronic device 20 is configured to communicate data by means of said first data communication block 22.

In one embodiment, the first data processing block 21 of the at least one user's portable electronic device 20 comprises the at least one first data processing unit 2.

In other words, the operations described above and also those subsequently provided for in the operation of the at least one first data processing unit 2 in accordance with different embodiments, are delegated to the at least one user's portable electronic device 20, in particular to the first data processing block 21.

According to one embodiment, as an alternative to the previous, the first data processing block 21 of the at least one user's portable electronic device 20 is distinct from the at least one first data processing unit 2.

In this embodiment, the first data processing block 21 of the at least one user's portable electronic device 20 is configured to communicate data to the at least one first data processing unit 2 via said first data communication block 22.

In this embodiment, from a functional point of view, the first data processing block 21 of the at least one user's portable electronic device 20 is configured to determine the value Q1 of amount of physical exercise performed by the user, as defined above.

Furthermore, the first data processing block 21 of the at least one user's portable electronic device 20 is configured to transmit to the at least one first data processing unit 2, via said first data communication block 22, the determined value Q1 of amount of physical exercise performed by the user.

Therefore, in this embodiment, the operation of determining the value Q1 of amount of physical exercise performed by the user is delegated to the first data processing block 21 of the at least one user's portable electronic device 20 (and not to the at least one first data processing unit 2), while all other operations described above and also subsequently provided for in the operation of the at least one first data processing unit 2 in accordance with different embodiments remain effectively the responsibility of the at least one first data processing unit 2.

According to one embodiment, in combination with any of the preceding ones in which the at least one user's portable electronic device 20 and the first data processing block 21 are provided and shown in figure 4, the at least one user's portable electronic device 20 comprises a respective first user interface module 23 operatively connected to the first data processing block 21 of the at least one user's portable electronic device 20.

In this embodiment, the at least one first data processing unit 2 is configured to send, based on the comparison between the determined value Q1 of amount of physical exercise and the set reference value R1, a first notification message N1 comprising the first information AP-1 or the second information AP-2.

In this embodiment, the first data processing block 21 is configured to provide the user with the first notification message N1 via the first user interface module 23.

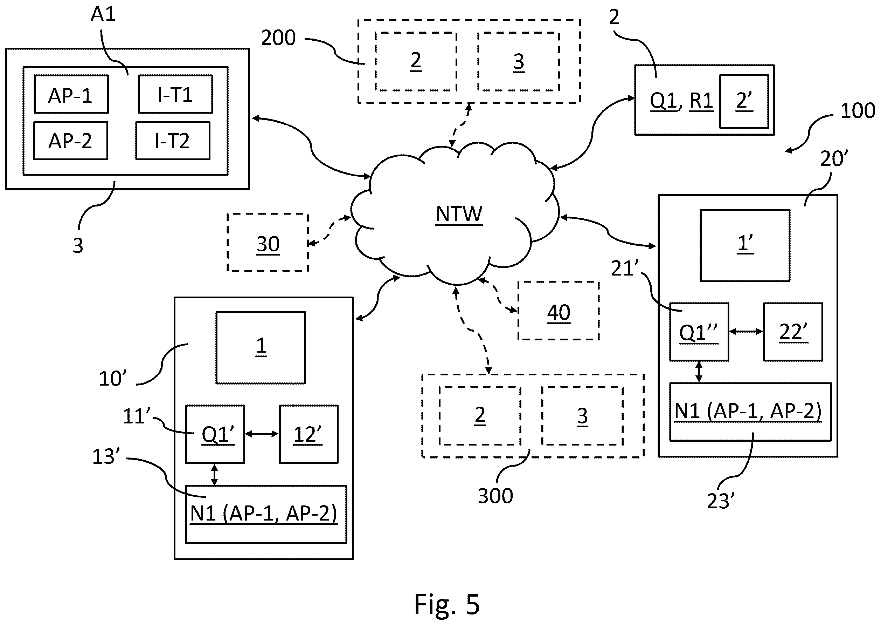

According to one embodiment, as an alternative to the preceding ones in which the exercise machine 10 comprising the first electronic assembly 1 is provided, or in which the at least one user's portable electronic device 20 comprising the first electronic assembly 1 is provided, and shown in figure 5, the system 100 comprises an exercise machine 10' operatively connected to the at least one first data processing unit 2.

Examples of exercise machine 10' employable in the embodiment of figure 5 are the same as those previously indicated with reference to the embodiment of figure 3.

The exercise machine 10' comprises said first electronic assembly 1 for tracking the physical activity performed by the user.

The physical exercise for which the amount value Q1 is determined comprises, at least in part, the physical exercise performed by the user using the exercise machine 10'.

The first electronic assembly 1 employable in the exercise machine 10' of the embodiment of figure 5 is the same as the first electronic assembly 1 already described above with reference to the exercise machine 10 of the embodiments described with reference to figure 3.

In this embodiment, the system 100 comprises at least one user's portable electronic device 20' operatively connected to the at least one first data processing unit 2.

The at least one user's portable electronic device 20' comprises a second electronic assembly 1' for tracking the physical activity performed by the user.

The second electronic assembly 1' for tracking the physical activity performed by the user employable in this case will be described below.

In this embodiment, the physical exercise for which the value Q1 of amount is determined comprises, at least in part, also the physical exercise performed by the user using the at least one user's portable electronic device 20', and thus not using the exercise machine 10'.

In greater detail, in this embodiment, the at least one user's portable electronic device 20' may be a device wearable by the user such as, for example, a bracelet or a smartwatch, or a portable device associable with the user such as, for example, a smartphone that can be associated with a part of the user's body (for example, a smartphone housed in an armband of the user).

Therefore, in this embodiment, the physical exercise for which the amount value Q1 is determined may comprise, possibly in part, any physical exercise that can be performed by the user equipped with the at least one user's portable electronic device 20', worn or associated with the user, for example, running, walking, cycling, squats, jumps or any other bodyweight exercise.

In other words, in this embodiment, the physical exercise for which the amount value Q1 is determined may be performed using the exercise machine 10' (for example in a gym training session) and using the at least one user's portable electronic device 20' (for example in a further bodyweight training session, indoor or outdoor).

In this embodiment, the second electronic assembly 1' for tracking the physical activity performed comprises, in this case, one or more additional sensor modules adapted to perform measurements of one or more parameters relating to the at least one user's portable electronic device 20', associable with the user's movement, and at least one additional data processing module adapted to determine additional data representative of the physical activity performed by the user (for example, movement data of the user) on the basis of the measurements performed by said one or more additional sensor modules.

For example, the at least one additional data processing module of the second electronic assembly 1' is configured to determine the following parameters relating to the at least one user's portable electronic device 20' by means of the measurements provided by the one or more sensor modules:
- the distance covered by the user of the second electronic assembly 1', thus by the user, during the performance of the physical activity, by means of the position (and possibly its variation over time) detected for example by a GPS module;
- the time taken by the user to cover such distance;
- the number of repetitions of a bodyweight exercise (for example: squats, jumps, and so on) during the performance of the physical activity, by means of the measurement of the acceleration to which the second electronic assembly 1' is subjected as a result of the user's movement, for example through the measurements provided by an accelerometer;
- the speed of the user along a route, by means of the measurement of the acceleration to which the first electronic assembly 1 is subjected as a result of the user's movement, for example through the measurements provided by an accelerometer.

According to one embodiment, in combination with the previous one and shown in figure 5, the exercise machine 10' comprises a respective first data processing block 11' and a respective first data communication block 12' operatively connected to the first data processing block 11'.

The first data processing block 11' of the exercise machine 10' is configured to communicate data via said first data communication block 12'.

In this embodiment, the at least one user's portable electronic device 20' comprises a respective second data processing block 21' and a respective second data communication block 22' operatively connected to the second data processing block 21'.

The second data processing block 21' is configured to communicate data via said second data communication block 22'.

According to different embodiments, in combination with the previous one, the at least one data processing unit 2 may correspond to one of the first data processing block 11' of the exercise machine 10' and the second data processing block 21' of the at least one user's portable electronic device 20', or be distinct from both the first data processing block 11' of the exercise machine 10' and the second data processing block 21' of the at least one user's portable electronic device 20'.

If the at least one data processing unit 2 corresponds to one of the first data processing block 11' of the exercise machine 10' and the second data processing block 21' of the at least one user's portable electronic device 20', the operations described above and also those subsequently provided for in the operation of the at least one first data processing unit 2 in accordance with different embodiments are delegated to the data processing block to which the at least one first data processing unit 2 corresponds.

**In** this case, the at least one first data processing unit 2 is configured to determine the value Q1 of the amount of physical exercise as a function of the data representative of the physical activity performed by the user tracked by the first electronic assembly 1 and the additional data representative of the physical activity performed by the user tracked by the second electronic assembly 1'.

**If** the at least one first data processing unit 2 is distinct from both the first data processing block 11' of the exercise machine 10' and the second data processing block 21' of the at least one user's portable electronic device 20', the at least one first data processing unit 2 is configured to determine the value Q1 of the amount of physical exercise as a function of a first value Q1' of the amount of physical exercise determined (and communicated via the first data communication block 12') by the first data processing block 11' based on the data representative of the physical activity performed by the user tracked by the first electronic assembly 1 (on the exercise machine 10') and a second value Q1" of the amount of physical exercise determined (and communicated via the second data communication block 22') by the second data processing block 21' based on the additional data representative of the physical activity performed by the user tracked by the second electronic assembly 1' (on the at least one second portable electronic device 20').

Therefore, in this embodiment, the operation of determining the value Q1 of the amount of physical exercise performed by the user is partially delegated to the data processing blocks 11' and 21' (and not entirely to the at least one first data processing unit 2), whereas all the other operations described above and also those subsequently provided for in the operation of the at least one first data processing unit 2 in accordance with different embodiments remain effectively the responsibility of the at least one first data processing unit 2.

Alternatively, the at least one first data processing unit 2 is configured to determine the value Q1 of the amount of physical exercise as a function of the data representative of the physical activity performed by the user tracked by the first electronic assembly 1 and the additional data representative of the physical activity performed by the user tracked by the second electronic assembly 1'.

Therefore, in this case, the operations described above and also those subsequently provided for in the operation of the at least one first data processing unit 2 in accordance with different embodiments are always delegated to the at least one first data processing unit 2.

According to one embodiment, in combination with the previous ones in which the exercise machine 10' and the respective first data processing block 11' and the at least one user's portable electronic device 20' and the respective first data processing block 21' are present, shown in figure 5, the exercise machine 10' comprises a respective first user interface module 13' operatively connected to the first data processing block 11' of the exercise machine 10' and the at least one user's portable electronic device 20' comprises a respective second user interface module 23' operatively connected to the second data processing block 21'.

In this embodiment, the at least one first data processing unit 2 is configured to send, based on the comparison between the determined value Q1 of the amount of physical exercise and the set reference value R1, a first notification message N1 comprising the first information AP-1 or the second information AP-2.

In this embodiment, the first data processing block 11' of the exercise machine 10' is configured to provide the user with the first notification message N1 via the first user interface module 13' of the exercise machine 10'.

In this embodiment, the second data processing block 21' of the at least one user's portable electronic device 20' is configured to provide the user with the first notification message N1 via the second user interface module 23'.

According to one embodiment, in combination with any of the preceding, when providing the user and/or personnel qualified to issue an authorization to purchase the drug with the generated first information AP-1 representative of the authorization for the user to take the next drug dose or when providing the user and/or personnel qualified to issue an authorization to purchase the drug with the second information AP-2 representative of a non-authorization for the user to take the next drug dose, the at least one first data processing unit 2 is configured to store the generated first information AP-1 representative of the authorization for the user to take the next drug dose or the generated second information AP-2 representative of the non-authorization for the user to take the next drug dose in the at least one first memory unit 3 in a first reserved area A1 for consultation by the user and/or personnel qualified to issue an authorization to purchase the drug.

According to one embodiment, in combination with any of the preceding and shown with dashed lines in figures 2-5, the system 100 further comprises a remote electronic processor 200 (or cloud server) and/or a further remote electronic processor 300 (or cloud server).

The first remote electronic processor 200 may be, for example, a cloud server of a fitness centre, while the second remote electronic processor 300 is, for example, a cloud server of a medical or healthcare centre.

According to one embodiment, in combination with the previous ones, the at least one first memory unit 3 may be included in the remote electronic processor 200 or in the further remote electronic processor 300.

In one embodiment, in combination with any of the preceding ones in which the remote electronic processor 200 and/or the further remote electronic processor 300 are provided, as an alternative to any of the preceding ones in which the at least one first data processing unit 2 is included in the exercise machine 10, in the exercise machine 10', in the at least one user's portable electronic device 20 or in the at least one user's portable electronic device 20', also shown with dashed lines in figures 2-5, the at least one first data processing unit 2 may be included in the remote electronic processor 200 or in the further remote electronic processor 300.

According to a further embodiment, in combination with any of the preceding ones in which the first reserved area A1 for consultation is provided in the at least one first memory unit 3, the at least one first data processing unit 2 is configured to determine and store in the at least one first memory unit 3 in the first reserved area A1 for consultation by the user and/or by personnel qualified to issue an authorization to purchase the drug an information I-T1 representative of the difference between the set reference value R1 and the determined value Q1 of the amount of physical exercise performed.

In this way, it is possible to monitor the progress status of the determination of the value Q1 of the amount of physical exercise with respect to the set reference value R1.

According to a further embodiment, in combination with any of the preceding ones in which the first reserved area A1 for consultation is provided in the at least one first memory unit 3, the at least one first data processing unit 2 is configured to determine and store in the at least one first memory unit 3 in the first reserved area A1 for consultation by the user and/or personnel qualified to issue an authorization to purchase the drug an additional information I-T2 representative of a target time period to reach the set reference value R1 of the amount of physical exercise.

According to one embodiment, in combination with the preceding ones in which the exercise machine 10 is present, the exercise machine 10 is configured to access, upon user command, the first reserved area A1 for consultation.

According to one embodiment, as an alternative to the previous ones and in combination with those in which the at least one user's portable electronic device 20 is provided, the at least one user's portable electronic device 20 is configured to access, upon user command, the first reserved area A1 for consultation.

According to one embodiment, as an alternative to the preceding ones and in combination with those in which the exercise machine 10' and the at least one user's portable electronic device 20' are provided, the at least one exercise machine 10' and/or the at least one user's portable electronic device 20' are configured to access, upon user command, the first reserved area A1 for consultation.

According to one embodiment, as an alternative to the preceding ones and shown with dashed lines in the figures, the system 100 further comprises a further first user's electronic device 30 configured to access, upon user command, the first reserved area A1 for consultation.

According to one embodiment, in combination with any of the preceding and shown with dashed lines in the figures, the system 100 further comprises at least one second electronic device 40 of personnel qualified to issue an authorization to purchase the drug, configured to access, upon command, the first reserved area A1 for consultation.

According to one embodiment, in combination with any of the preceding ones, the at least one first data processing unit 2 is configured to monitor the provision of drug doses to be taken by the user based on a count of each generated information representative of an authorization for the user to take a next drug dose.

This count may be performed directly by the at least one first data processing unit 2 or by the remote electronic processor 200 (if present) or by the further remote electronic processor 300 (if present).

According to one embodiment, in combination with any of the preceding in which the exercise machine 10 or the exercise machine 10' is provided, the exercise machine 10 (10') may be an exercise machine usable by a user for performing strength exercises, an exercise machine for performing cardiovascular exercises, or an exercise machine for performing combined cardiovascular and strength exercises.

According to one embodiment, in combination with any of those previously described, the determined value Q1 of the amount of physical exercise and the set reference value R1 are expressed in METh.

In this regard, in a first embodiment, the amount of physical exercise performed by the user can indeed be actually quantified using MET on an hourly basis (METh, MET per hour).

A MET (Metabolic Equivalent of Task) is an absolute and timeless unit of measurement by which the intensity of physical exercise performed by a user can be expressed.

For example, at rest, the intensity of physical exercise of a user is equal to 1 MET.

As the intensity of physical exercise performed by the user increases (for example, walking, running, jumping), the number of METs increases.

The indication of MET on an hourly basis allows for transitioning from intensity of physical exercise to volume of exercise, i.e., the amount of physical exercise performed by the user.

For example:
- if a user sleeps for one hour, such user will have accumulated 1 METh;
- if a user runs for one hour at 10 km/h, corresponding to an intensity of 10,5 MET, such user will have accumulated 10,5 METh;
- if a user runs for half an hour at 10 km/h, corresponding to an intensity of 10.5 MET, such user will have accumulated 10,5 x 30 / 60 METh;
- if a user trains by lifting weights for 45 minutes (this activity can be quantified at an average intensity of 5 MET as reported in literature references
- for example, the reference Ainsworth BE, Haskell WL, Herrmann SD, et al.

*"*Compendium of physical activities: classification of energy costs of human physical activities, Med Sci Sports Exerc. 1993; 25(1): 71-80*"* and subsequent updates), such user will have accumulated 5 x 45 / 60 = 3.75 METh.

Each type of physical exercise corresponds to a value of physical exercise intensity in MET, as reported in various scientific publication databases.

Therefore, knowing the execution time (or duration) of the exercise, it is possible to obtain the volume of physical exercise performed by a user by multiplying MET by duration.

It should be noted that the use of METh as a reference for quantifying the amount of physical exercise performed by the user is equivalent to the use of the unit for the measurement of calories, since these can be converted into METh by dividing them by the user's weight (expressed in kg).

The amount of physical exercise performed by the user can be detected (tracked) in different ways and with various electronic devices that use different technologies.

According to a first mode, an exercise machine such as, for example, a treadmill, a bike, a chest press, may be equipped with the first electronic tracking assembly 1 of the physical activity performed by the user, as described previously with reference to the embodiments in which the exercise machine comprises the first electronic tracking assembly 1 of the physical activity performed by the user, to track the data representative of the user's physical activity.

In more detail, one or more sensor modules of the first electronic assembly 1 perform measurements of one or more operating parameters of the exercise machine and at least one data processing module of the first electronic assembly, through dedicated software, determines the aforesaid data representative of the physical activity performed by the user (for example, movement data of the user on the exercise machine 10 or 10') on the basis of the measurements performed by said one or more sensor modules.

On the basis of the aforesaid data representative of the physical activity performed by the user, it is possible to determine a value Q1 of the amount of physical exercise performed by the user (expressed in METh or calories).

According to a further mode, useful for example in the case of physical activity performed outdoors such as running or walking, the tracking of training data may be performed by means of the at least one portable electronic device 20 of the user (possibly also wearable), such as a smartphone or a smartwatch, equipped with the first electronic assembly 1 or with the second electronic assembly 1' for tracking the physical activity performed by the user, as described previously with reference to the embodiments in which the at least one portable electronic device 20 of the user comprises the first electronic assembly 1 or the second electronic assembly 1' for tracking the physical activity performed by the user, to track the data representative of the user's physical activity.

In more detail, one or more sensor modules of the first electronic assembly 1 (or of the second electronic assembly 1') perform measurements of one or more parameters related to the user's portable electronic device, associable with the user's movement, and at least one data processing module is adapted to determine the aforesaid data representative of the physical activity performed by the user (for example, user's movement data) on the basis of the measurements performed by said one or more sensor modules.

On the basis of the aforesaid data representative of the physical activity performed by the user, it is possible to determine a value Q1 of the amount of physical exercise performed by the user (expressed in METh or calories).

More specifically, with reference to one of the embodiments described with reference to figure 3, in the case in which the exercise machine 10, operatively connected to the at least one first data processing unit 2, is configured to communicate data, for example through the data communication network NTW, with the at least one first data processing unit 2, the at least one exercise machine 10 is capable of communicating to the at least one first data processing unit 2 the determined value Q1 of the amount of physical exercise performed by the user expressed as a number of METh.

In fact, for each exercise machine, based on the operating parameters of the particular type of machine (for example, speed and inclination for the treadmill, resistance and speed of rotation of the pedals for the stationary bike, and so on), it is possible to determine the corresponding value of exercise intensity in MET and, based on the duration of the physical exercise with those operating parameters, the corresponding value of volume in METh.

The correspondence between the operating parameters of the exercise machine and MET results from tables and/or formulas established by scientific literature.

According to another example, in the case in which the physical exercise is within a set training program provided by a software application usable by the user, for example through their smartphone or through the at least one exercise machine 10, the user, at the end of the physical exercise performed, may manually select, via the software application, a command corresponding to the state "physical exercise performed."

In this case, information representative of the training data, and therefore also the value of the amount of physical exercise performed by the user expressed in MET, has already been tracked and stored via the software application.

With the estimate of the duration of the physical exercise (training time), the software application is able to make available to the at least one first data processing unit 2 the determined value Q1 of the amount of physical exercise performed by the user expressed as a number of METh.

According to a further example, in which the physical exercise (for example, a run or a walk) is performed by the user outdoors and the user has loaded and is running a software application on their smartphone, such software application, via the geolocation system (for example, GPS) of the smartphone, is capable of determining the value of physical exercise intensity (expressed in MET) and, by measuring the duration of the physical exercise, determining the corresponding value Q1 of the amount of physical exercise performed by the user expressed as a number of METh to be made available to the at least one first data processing unit 2.

With regard to the set reference value R1, the set reference value R1 is representative of the threshold upon reaching which it is necessary to authorise the user to take a subsequent dose of the drug.

In this manner, the user is guaranteed to be able to maintain the benefit of the drug during the performance of physical exercise, i.e., the enhancement of the effect of the drug and/or the reduction of a possible side effect of the drug during the performance of physical exercise.

This value is the result of an evaluation carried out by a physician and depends on the drug or the pathology of interest.

The set reference value R1 may be a minimum amount of physical exercise such that a user is considered "Active".

For example, a possible classification may be as follows:
- value of the amount of physical exercise < 8 METh per week: user considered "slightly active";
- value of the amount of physical exercise between 8 METh per week and 16,5 METh per week: user considered "active";
- value of the amount of physical exercise > 16,5 METh per week: user considered "very active".

The weekly value may be optionally scaled on a daily basis.

It should be noted that the set reference value R1 may be periodically re-evaluated by the physician at the end of a verification period which may be, for example, one month.

According to one embodiment, in combination with any of the preceding ones, if the determined value Q1 of the amount of physical exercise is greater than or equal to the set reference value R1, the at least one first data processing unit 2 is configured to provide the user and/or a person authorised to grant an authorisation to purchase the drug with the generated first information AP-1 representative of the authorisation to take the next dose of the drug only at the end of a set minimum interval of time, elapsed from the first point in time when the drug dose was taken by the user, before being able to take the subsequent drug dose.

This set minimum interval of time depends on the type of drug and on the time frequency envisaged for taking two successive doses of the drug (for example, once a week, once every two weeks, and so on).

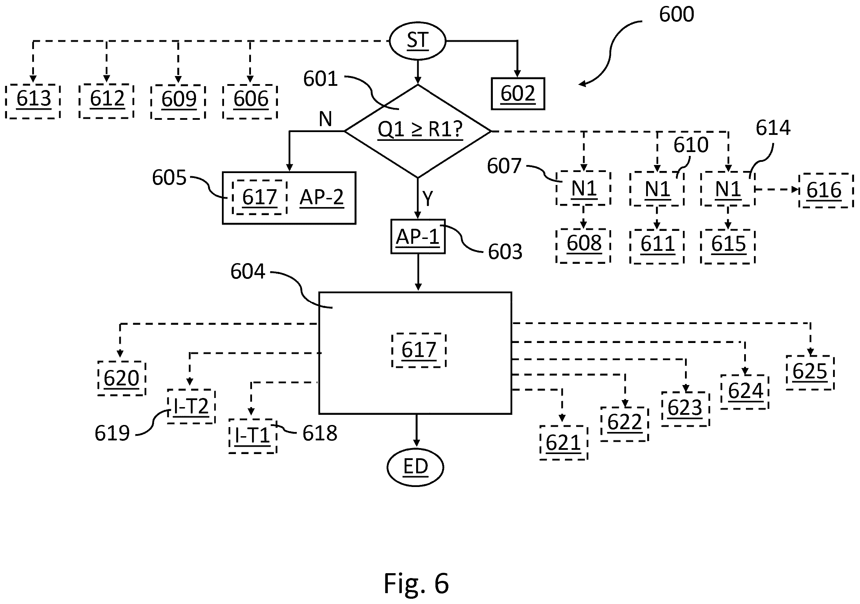

With reference to figure 6, a method 600 for managing the authorisation to proceed with an intake of a drug dose by a user as a function of the physical activity performed by the user is now described, hereinafter also referred to as management method or simply method, in accordance with the present invention.

The definition of drug has been provided previously.

The method 600 comprises a symbolic step of starting ST.

The method 600 also comprises a step of comparing 601, by at least one first data processing unit 2 operatively connected to at least one first electronic assembly 1 for tracking the physical activity performed by the user, a value Q1 of the amount of physical exercise performed by the user, from a first point in time in which a drug dose was taken by the user, with a set reference value R1.

The value Q1 of the amount of physical exercise performed by the user has been determined, from the first point in time in which a drug dose was taken by the user, based on data representative of the physical activity performed by the user tracked by said first electronic assembly 1 for tracking the physical activity performed by the user.

The at least one first data processing unit 2, the first electronic assembly 1, and the amount of physical exercise have been previously described.

The method 600 also comprises steps of storing 602, by the at least one first data processing unit 2, in at least one first memory unit 3 operatively connected to the at least one first data processing unit 2, the determined value Q1 of the amount of physical exercise performed by the user.

The method 600 further comprises the steps of:
(Y) if the determined value Q1 of the amount of physical exercise is greater than or equal to the set reference value R1:
- generating 603, by said at least one first data processing unit 2, a first information AP-1 representative of an authorisation for the user to take the next drug dose;
- providing 604, by said at least one first data processing unit 2, to the user and/or to a person authorised to grant an authorisation to purchase the drug the generated first information AP-1 representative of the authorisation for the user to take the next dose of the drug.

Therefore, the at least one first data processing unit 2, in the event that the determined value Q1 representative of the amount of physical exercise performed by the user is greater than or equal to the set reference value R1, generates the first information AP-1 representative of an authorisation for the user to take the next dose of the drug since it is necessary for the user to take a subsequent dose of the drug, considering that the outcome of the comparison indicates that the beneficial effect of the previous dose of the drug has worn off and/or there is still a risk of a side effect of the drug during the performance of physical exercise.

The method 600 also comprises the steps of:
(N) if the determined value Q1 of the amount of physical exercise is less than the set reference value R1:
- generating and providing 605 to the user and/or to a person authorised to grant an authorisation to purchase the drug, by said at least one first data processing unit 2, a second information AP-2 representative of a non-authorisation for the user to take the next dose of the drug or not generating any first information AP-1 representative of an authorisation for the user to take the next dose of the drug.

In other words, the at least one first data processing unit 2, in the event that the determined value Q1 representative of the amount of physical exercise performed by the user is less than the set reference value R1, generates a second information AP-2 representative of a non-authorisation for the user to take the next dose of the drug or does not generate any first information AP-1 representative of an authorisation for the user to take the next dose of the drug, since the user has not met the necessary condition to obtain authorisation to take a subsequent dose of the drug or that the dose of the drug must be varied.

With further reference to figure 6, the method 600 also comprises a symbolic step of ending ED.

According to one embodiment, the value Q1 of the amount of physical exercise performed by the user is determined by the at least one first data processing unit 2.

According to one embodiment, a exercise machine 10 is operatively connected to the at least one first data processing unit 2.

The exercise machine 10 has already been described previously.

In this embodiment, the exercise machine 10 comprises said first electronic assembly 1 for tracking the physical activity performed by the user.

In this regard, the first electronic assembly 1 employable in this case has been previously described.

The physical exercise of which the amount value Q1 is determined is at least partly performed by the user using the exercise machine 10.

According to one embodiment, in combination with the preceding one, the exercise machine 10 comprises a respective first data processing block 11 and a respective first data communication block 12 operatively connected to the first data processing block 11.

In this embodiment, shown in figure 3 with dashed lines, the method 600 comprises a step of communicating data 606, by the first data processing block 11 of the exercise machine 10, via said first data communication block 12.

In one embodiment, the first data processing block 11 of the exercise machine 10 comprises the at least one first data processing unit 2.

In other words, the operations, described previously and also subsequently provided for in the method according to various embodiments, are entrusted to the exercise machine 10, in particular to the first data processing block 11.

According to one embodiment, as an alternative to the preceding one, the first data processing block 11 of the exercise machine 10 is distinct from the at least one first data processing unit 2.

In this embodiment, the first data processing block 11 of the exercise machine 10 is operatively connected to the at least one first data processing unit 2.

In this embodiment, the step of communicating data 606 via said first data communication block 12 is carried out by the first data processing block 11 of the exercise machine 10 to communicate data to the at least one first data processing unit 2.

In this embodiment, from a functional point of view, the value Q1 of the amount of physical exercise performed by the user, defined previously, is determined by the first data processing block 11 of the exercise machine 10.

Furthermore, the step of communicating data 606 via said first data communication block 12 is carried out by the first data processing block 11 of the exercise machine 10 to transmit to the at least one first data processing unit 2 the determined value Q1 of the amount of physical exercise performed by the user.

Therefore, in this embodiment, the operation of determining the value Q1 of the amount of physical exercise performed by the user is entrusted to the first data processing block 11 of the exercise machine 10 (and not to the at least one first data processing unit 2) while all other operations, described previously and also subsequently provided for in the operation of the at least one first data processing unit 2 in accordance with various embodiments, remain actually entrusted to the at least one first data processing unit 2.

According to one embodiment, in combination with any of the preceding ones in which the exercise machine 10 is provided, the exercise machine 10 comprises a respective first user interface module 13 operatively connected to the first data processing block 11 of the exercise machine 10.

In this embodiment, shown with dashed lines in figure 6, the method 600 also comprises a step of sending 607, by the at least one first data processing unit 2, based on the comparison between the determined value Q1 of the amount of physical exercise and the set reference value R1, a first notification message N1 comprising the first information AP-1 or the second information AP-2.

In this embodiment, the method 600 also comprises a step of providing 608 to the user, by the first data processing block 11, the first notification message N1 via the first user interface module 13 of the exercise machine 10.

According to one embodiment, in combination with any of the preceding ones in which the exercise machine 10 is not provided, at least one portable electronic device 20 of the user is operatively connected to the at least one first data processing unit 2.

The at least one portable electronic device 20 of the user comprises said first electronic assembly 1 for tracking the physical activity performed by the user.

The first electronic assembly 1 for tracking the physical activity performed by the user employable in this case has been previously described.

In this embodiment, the physical exercise of which the amount value Q1 is determined is at least partly performed by the user using the at least one portable electronic device 20 of the user.

According to one embodiment, in combination with the preceding one, the at least one portable electronic device 20 of the user comprises a respective first data processing block 21 and a respective first data communication block 22 operatively connected to the first data processing block 21.

In this embodiment, shown with dashed lines in figure 6, the method 600 also comprises a step of communicating data 609, carried out by the first data processing block 21 of the at least one portable electronic device 20, via said first data communication block 22.

In one embodiment, the first data processing block 21 of the at least one portable electronic device 20 of the user comprises the at least one first data processing unit 2.

In other words, the operations, described previously and also subsequently provided for in the method according to various embodiments, are entrusted to the at least one portable electronic device 20 of the user, in particular to the first data processing block 21.

According to one embodiment, as an alternative to the preceding one, the first data processing block 21 of the at least one portable electronic device 20 of the user is distinct from the at least one first data processing unit 2.

In this embodiment, the step of communicating data 609 via said first data communication block 22 is carried out by the first data processing block 21 of the at least one portable electronic device 20 of the user to communicate data to the at least one first data processing unit 2.

In this embodiment, from a functional point of view, the value Q1 of the amount of physical exercise performed by the user, defined previously, is determined by the first data processing block 21 of the at least one portable electronic device 20 of the user.

Furthermore, the step of communicating data 609 via said first data communication block 22 is carried out by the first data processing block 21 of the at least one portable electronic device 20 of the user to transmit from the at least one portable electronic device 20 of the user the determined value Q1 of the amount of physical exercise performed by the user.

Therefore, in this embodiment, the operation of determining the value Q1 of the amount of physical exercise performed by the user is entrusted to the first data processing block 21 of the at least one portable electronic device 20 of the user (and not to the at least one first data processing unit 2) while all other operations, described previously and also subsequently provided for in the operation of the at least one first data processing unit 2 in accordance with various embodiments, remain actually entrusted to the at least one first data processing unit 2.

According to one embodiment, in combination with the preceding one, the at least one portable electronic device 20 of the user comprises a respective first user interface module 23 operatively connected to the first data processing block 21.

In this embodiment, shown with dashed lines in figure 6, the method 600 also comprises a step of sending 610, by the at least one first data processing unit 2, based on the comparison between the determined value Q1 of the amount of physical exercise and the set reference value R1, a first notification message N1 comprising the first information AP-1 or the second information AP-2.

The method 600 also comprises a step of providing 611 to the user, by the first data processing block 21, the first notification message N1 via the first user interface module 23.

According to one embodiment, as an alternative to the preceding one in which the exercise machine 10 comprising the first electronic assembly 1 is provided or in which the at least one portable electronic device 20 comprising the first electronic assembly 1 is provided, an exercise machine 10' is operatively connected to the at least one first data processing unit 2.

The exercise machine 10' comprises said first electronic assembly 1 for tracking the physical activity performed by the user.

The physical exercise of which the amount value Q1 is determined is at least partly performed by the user using the exercise machine 10'.

In this embodiment, at least one portable electronic device 20' of the user is operatively connected to the at least one first data processing unit 2.

The at least one portable electronic device 20' of the user comprises a second electronic assembly 1' for tracking the physical activity performed by the user.

The second electronic assembly 1' for tracking the physical activity performed by the user employable in this case has been previously described.

In this embodiment, the physical exercise of which the amount value Q1 is determined includes, optionally in part, also the physical exercise performed by the user using the at least one portable electronic device 20' of the user, thus not using the exercise machine 10'.

The at least one portable electronic device 20' of the user has been described previously.

Therefore, in this embodiment, the physical exercise of which the amount value Q1 is determined can be performed using the exercise machine 10' (for example in a training session at the gym) and using the at least one portable electronic device 20' of the user (for example in an additional bodyweight training session, indoor or outdoor).

According to one embodiment, in combination with the preceding one, the exercise machine 10' comprises a respective first data processing block 11' and a respective first data communication block 12' operatively connected to the first data processing block 11'.

In this embodiment, shown with dashed lines in figure 6, the method 600 comprises a step of communicating data 612, by said first data processing block 11', via said first data communication block 12'.

In this embodiment, the at least one portable electronic device 20' of the user comprises a respective second data processing block 21' and a respective second data communication block 22' operatively connected to the second data processing block 21'.

The method 600 further comprises a data communication step 613, carried out by the second data processing block 21', via said second data communication block 22'.

According to various embodiments, in combination with the preceding one, the at least one data processing unit 2 may correspond to one of the first data processing block 11' of the exercise machine 10' and the second data processing block 21' of the at least one portable electronic device 20' or may be distinct from both the first data processing block 1 1' of the exercise machine 10' and the second data processing block 21' of the at least one portable electronic device 20'.

In the case in which the at least one data processing unit 2 corresponds to one of the first data processing block 11' of the exercise machine 10' and the second data processing block 21' of the at least one portable electronic device 20', the operations described previously and also subsequently provided for in the method according to various embodiments are entrusted to the data processing block to which the at least one first data processing unit 2 corresponds.

In this case, the at least one first data processing unit 2 is configured to determine the value Q1 of the amount of physical exercise based on data representative of the physical activity performed by the user tracked by the first electronic assembly 1 and additional data representative of the physical activity performed by the user tracked by the second electronic assembly 1'.

In the case in which the at least one first data processing unit 2 is distinct from both the first data processing block 11' of the exercise machine 10' and the second data processing block 21' of the at least one portable electronic device 20', the at least one first data processing unit 2 is configured to determine the value Q1 of the amount of physical exercise as function of a first value Q1' of the amount of physical exercise determined by the first data processing block 11' as function of data representative of the physical activity performed by the user tracked by the first electronic assembly 1 and a second value Q1" of the amount of physical exercise determined by the second data processing block 21' as function of the additional data representative of the physical activity performed by the user tracked by the second electronic assembly 1'.

Therefore, in this embodiment, the operation of determining the value Q1 of the amount of physical exercise performed by the user is entrusted in part to the data processing blocks 11' and 21' (and not entirely to the at least one first data processing unit 2) while all other operations, previously described and also subsequently provided for in the method according to various embodiments, remain effectively entrusted to the at least one first data processing unit 2.

Alternatively, the value Q1 of the amount of physical exercise is determined by the at least one first data processing unit 2 as function of data representative of the physical activity performed by the user tracked by the first electronic assembly 1 and the additional data representative of the physical activity performed by the user tracked by the second electronic assembly 1'.

Therefore, in this case, the operations previously described and also subsequently provided for in the method according to various embodiments are always entrusted to the at least one first data processing unit 2.

According to one embodiment, in combination with the preceding one and shown with dashed lines in figure 6, the method 600 also comprises a step of sending 614, by the at least one first data processing unit 2, based on the comparison between the determined value Q1 of the amount of physical exercise and the set reference value R1, a first notification message N1 comprising the first information AP-1 or the second information AP-2.

In this embodiment, the exercise machine 10' comprises a respective first user interface module 13' operatively connected to the first data processing block 11'.

The method 600 comprises a step of providing 615 to the user, carried out by the first data processing block 11', the first notification message N1 via the first user interface module 13'.

The at least one portable electronic device 20' of the user comprises a respective second user interface module 23' operatively connected to the second data processing block 21'.

The method 600 comprises a step of providing 616 to the user, carried out by the second data processing block 21', the first notification message N1 via the second user interface module 23'.

In one embodiment, in combination with any of the preceding ones and shown with dashed lines in figure 6, the step of providing 604 to the user and/or to personnel qualified to authorise the purchase of the drug the generated first information AP-1 representative of the authorisation for the user to take the next drug dose, or the step of generating and providing 605 to the user and/or to personnel qualified to authorise the purchase of the drug the second information AP-2 representative of a non-authorisation for the user to take the next drug dose, comprises a step of storing 617, by the at least one first data processing unit 2, the generated first information AP-1 representative of the authorisation for the user to take the next drug dose or the generated second information AP-2 representative of the non-authorisation for the user to take the next drug dose in the at least one first memory unit 3 in a first reserved consultation area A1 for the user and/or for the personnel qualified to authorise the purchase of the drug.

According to a further embodiment, in combination with any of the preceding, the method 600 comprises a step of determining and storing 618, by the at least one first data processing unit 2, in the at least one first memory unit 3 in the first reserved consultation area A1 for the user and/or for personnel qualified to authorise the purchase of the drug, information I-T1 representative of the difference between the set reference value R1 and the determined value Q1 of the amount of physical exercise performed.

In this way, it is possible to monitor the progress of the determination of the value Q1 of the amount of physical exercise with respect to the set reference value R1.

According to a further embodiment, in combination with any of the preceding, the method 600 comprises a step of determining and storing 619, by the at least one first data processing unit 2, in the at least one first memory unit 3 in the first reserved consultation area A1 for the user and/or the personnel qualified to authorise the purchase of the drug, additional information I-T2 representative of a target time period to reach the set reference value of the amount of physical exercise.

According to one embodiment, in combination with any of the preceding ones in which the exercise machine 10 is provided, the method 600 comprises a step of accessing 620, by the exercise machine 10, upon user command, the first reserved consultation area A1.

According to one embodiment, as an alternative to the previous one and in combination with those in which the at least one portable electronic device 20 of the user is provided, the method 600 comprises a step of accessing 621, by the at least one portable electronic device 20 of the user, upon user command, the first reserved consultation area A1.

According to one embodiment, as an alternative to the previous one and in combination with those in which the exercise machine 10' and the at least one portable electronic device 20' of the user are provided, the method 600 comprises a step of accessing 622, by the at least one exercise machine 10' and/or the at least one portable electronic device 20' of the user, upon user command, the first reserved consultation area A1.

According to one embodiment, as an alternative to the previous ones, shown with dashed lines in figure 6, the method 600 comprises a step of accessing 623, by an additional first electronic device 30 of the user, upon user command, the first reserved consultation area A1.

According to one embodiment, in combination with any of the preceding ones, shown with dashed lines in figure 6, the method 600 comprises a step of accessing 624, by at least one second electronic device 40 of the personnel qualified to authorise the purchase of the drug configured to access, upon user command, the first reserved consultation area A1.

According to one embodiment, in combination with any of the preceding ones and shown with dashed lines in figure 6, the method 600 comprises a step of monitoring 625, by the at least one first data processing unit 2, the supply of medication doses to be taken by the user based on a count of each generated information representative of an authorisation for the user to take a subsequent drug dose.

This count may be performed directly by the at least one first data processing unit 2 or by the remote electronic processor 200 (if present) or by the additional remote electronic processor 300 (if present).

According to one embodiment, in combination with any of the preceding ones in which the exercise machine 10 or the exercise machine 10' and said at least one second portable electronic device 20' are provided, the exercise machine 10 (10') comprises an exercise machine usable by the user for performing strength exercises, an exercise machine for performing cardiovascular exercises, or an exercise machine for performing combined cardiovascular and strength exercises.

According to one embodiment, in combination with any of those previously described, the determined value Q1 of the amount of physical exercise and the set reference value R1 are expressed in METh.

The METs and some examples of their determination have already been described above.

The set reference value R1 and some examples for its determination have been previously defined.

According to one embodiment, in combination with any of the preceding ones, if the determined value Q1 of the amount of physical exercise is greater than or equal to the set reference value R1, the step of providing 604 to the user and/or to personnel qualified to authorise the purchase of the drug the generated first information AP-1 representative of the authorisation for the user to take the next drug dose is carried out, by the at least one first data processing unit 2, only after a set minimum time interval has elapsed since the first time instant in which the drug dose was taken by the user, in order to be able to take the next drug dose.

This set minimum time interval depends on the type of drug and the planned time interval for the intake of two successive doses of the drug (e.g., once a week, once every two weeks, and so on).

With reference now to the aforementioned figures, an example of operation of the system according to the present invention is described.

A user takes a drug dose for type 2 diabetes and starts physical exercise on an exercise machine 10, belonging to the system 100, usable by the user for performing muscle-strengthening exercises.

At least one first data processing unit 2 operatively connected to the exercise machine 10 compares a value Q1 of the amount of physical exercise performed by the user, from a first time instant when a drug dose was taken by the user, with a set reference value R1.

The value Q1 of the amount of physical exercise performed by the user has been determined, from the first time instant in which a drug dose was taken by the user, as function of on data representative of the physical activity performed by the user traced by said first electronic assembly 1 for tracking the physical activity performed by the user.

The at least one first data processing unit 2 stores, in at least one first memory unit 3 operatively connected to the at least one first data processing unit 2, the determined value Q1 of the amount of physical exercise performed by the user.

If the determined value Q1 of the amount of physical exercise is greater than or equal to the set reference value R1, the at least one first data processing unit 2 generates first information AP-1 representative of an authorisation for the user to take the next drug dose and provides the user and/or a personnel qualified to authorise the purchase of the drug with the generated first information AP-1 representative of the authorisation for the user to take the next drug dose.

If the determined value Q1 of the amount of physical exercise is less than the set reference value R1, the at least one first data processing unit 2 generates and provides the user and/or personnel qualified to authorise the purchase of the drug with second information AP-2 representative of a non-authorisation for the user to take the next drug dose or does not generate any first information AP-1 representative of an authorisation for the user to take the next drug dose.

As can be seen, the object of the invention is fully achieved.

Indeed, the system and the related method according to the present invention make it possible to optimise the beneficial effect for the user, also avoiding any potential side effects caused by the drug, by controlling the drug dose taken by the user precisely as a function of the activity performed by the user, for example starting from the moment the drug dose was taken, in order to determine the most appropriate moment to take the next drug dose.

Furthermore, the system and the related method advantageously allow monitoring the supply of the drug in order to possibly determine the need to obtain a new authorisation for purchase.

In addition, it is evident that the determination of the value of the amount of physical exercise, performed based on data representative of the physical activity performed by the user traced by a first electronic assembly for tracking the physical activity performed by the user, is certainly much more accurate and therefore enables greater effectiveness of the information provided as output by the system and method according to the present invention.

Moreover, the possibility of optionally having a first assembly for tracking the physical activity performed by the user, for example during a training session performed on an exercise machine, and a second electronic group for tracking the physical activity performed by the user, for example during an additional training session performed outdoors or as bodyweight training, before or after the training session on the exercise machine, makes it possible to trace the physical activity performed by the user as comprehensively as possible, even across successive training sessions, thus obtaining an even more accurate determination of the value of the amount of physical exercise, which makes the information provided as output by the system and method according to the present invention even more effective and reliable.

To the embodiments of the system and method described above, a person skilled in the art, to meet contingent needs, may make modifications, adaptations, and replacements of elements with others that are functionally equivalent, without departing from the scope of the following claims. Each of the features described as belonging to a possible embodiment may be implemented independently of the other embodiments described.

## Claims

1. A system (100) for managing the authorization to proceed with an intake of a drug dose by a user as a function of the physical activity performed by the user, said system (100) comprising:
- a first electronic assembly (1) for tracking the physical activity performed by the user;
- at least one first data processing unit (2) operatively connected to the first electronic assembly (1) for tracking the physical activity performed by the user;
- at least one first memory unit (3) operatively connected to the at least one first data processing unit (2);
the at least one first data processing unit (2) is configured to:
- compare a value (Q1) of the amount of physical exercise performed by the user, starting from a first time instant in which a drug dose is taken by the user, with a set reference value (R1), the value (Q1) of the amount of physical exercise performed by the user having been determined, starting from the first time instant in which a drug dose is taken by the user, as a function of data representative of the physical activity performed by the user tracked by said first electronic assembly (1) for tracking the physical activity performed by the user;
- store in the at least one first memory unit (3) the determined value (Q1) of the amount of physical exercise performed by the user;
(Y) if the determined value (Q1) of the amount of physical exercise is greater than or equal to the set reference value (R1):
- generate a first information (AP-1) representative of an authorization for the user to take the next drug dose;
- provide the user and/or personnel responsible for issuing an authorization to purchase the drug with the generated first information (AP-1), representative of the authorization for the user to take the next drug dose;
(N) if the determined value (Q1) of the amount of physical exercise is less than the set reference value (R1):
- generate and provide the user and/or personnel responsible for issuing an authorization for the purchase of the drug with a second information (AP-2) representative of a non-authorization for the user to take the next drug dose or not to generate a first information (AP-1) representative of an authorization for the user to take the next drug dose.

2. The system (100) according to claim 1, wherein the at least one first data processing unit (2) is configured to determine the value (Q1) of the amount of physical exercise performed by the user.

3. The system (100) according to any one of the preceding claims, comprising an exercise machine (10) operatively connected to the at least one first data processing unit (2), the exercise machine (10) comprising said first electronic assembly (1) for tracking the physical activity performed by the user, the physical exercise for which the amount value (Q1) is determined being at least partially performed by the user using the exercise machine (10).

4. The system (100) according to claim 3, wherein the exercise machine (10) comprises a respective first data processing block (11) and a respective first data communication block (12) operatively connected to the first data processing block (11), said first data processing block (11) being configured to communicate data by means of said first data communication block (12).

5. The system (100) according to claim 4, wherein the first data processing block (11) of the exercise machine (100) comprises the at least one first data processing unit (2).

6. The system (100) according to claim 4, wherein the first data processing block (11) of the exercise machine (100) is distinct from the at least one first data processing unit (2), the first data processing block (11) of the exercise machine (10) being operatively connected to the at least one first data processing unit (2), the first data processing block (11) of the exercise machine (10) being configured to communicate data to the at least one first data processing unit (2) by means of said first data communication block (12), the first data processing block (11) of the exercise machine (10) being configured to determine the value (Q1) of the amount of physical exercise performed by the user, the first data processing block (11) of the exercise machine (10) being configured to transmit to the at least one first data processing unit (2), by means of said first data communication block (12), the determined value (Q1) of the amount of physical exercise performed by the user.

7. The system (100) according to any one of claims 4 to 6, wherein the exercise machine (10) comprises a respective first user interface module (13) operatively connected to the first data processing block (11) of the exercise machine (1), the at least one first data processing unit (2) being configured to send, based on the comparison of the determined value (Q1) of the amount of physical exercise with the set reference value (R1), a first notification message (N1) comprising the first information (AP-1) or the second information (AP-2), the first data processing block (11) being configured to provide the user with the first notification message (N1) by means of the first user interface module (13) of the exercise machine (10).

8. The system (100) according to any one of the preceding claims 1 or 2, comprising at least one user's portable electronic device (20) operatively connected to the at least one first data processing unit (2), said at least one user's portable electronic device (20) comprising said first electronic assembly (1) for tracking the physical activity performed by the user, the physical exercise for which the amount value (Q1) is determined being at least partially performed by the user using the at least one user's portable electronic device (20).

9. The system (100) according to claim 8, wherein the at least one user's portable electronic device (20) comprises a respective first data processing block (21) and a respective first data communication block (22) operatively connected to the first data processing block (21), said first data processing block (21) of the at least one user's portable electronic device (20) being configured to communicate data by means of said first data communication block (22).

10. The system (100) according to claim 9, wherein the first data processing block (21) of the at least one user's portable electronic device (20) comprises the at least one first data processing unit (2).

11. The system (100) according to claim 9, wherein the first data processing block (21) of the at least one user's portable electronic device (20) is configured to communicate data to the at least one first data processing unit (2) by means of said first data communication block (22), the first data processing block (21) of the at least one user's portable electronic device (20) being configured to determine the value (Q1) of the amount of physical exercise performed by the user, the first data processing block (21) of the at least one user's portable electronic device (20) being configured to transmit to the at least one first data processing unit (2), by means of said first data communication block (22), the determined value (Q1) of the amount of physical exercise performed by the user.

12. The system (100) according to any one of the preceding claims 9 to 11, wherein the at least one user's portable electronic device (20) comprises a respective first user interface module (23) operatively connected to the first data processing block (21), the at least one first data processing unit (2) being configured to send, based on the comparison of the determined value (Q1) of the amount of physical exercise with the set reference value (R1), a first notification message (N1) comprising the first information (AP-1) or the second information (AP-2), the first data processing block (21) being configured to provide the user with the first notification message (N1) by means of the first user interface module (23).

13. The system (100) according to any one of claims 1 or 2, comprising:
- an exercise machine (10') operatively connected to the at least one first data processing unit (2), the exercise machine (10') comprising said first electronic assembly (1) tracking the physical activity performed by the user, the physical exercise for which the amount value (Q1) is determined comprising, at least partially, the physical exercise performed by the user using the exercise machine (10');
- at least one user's portable electronic device (20') operatively connected to the at least one first data processing unit (2), said at least one user's portable electronic device (20') comprising a second electronic assembly (1') tracking the physical activity performed by the user, the physical exercise for which the amount value (Q1) is determined comprising, at least partially, the physical exercise performed by the user using the at least one user's portable electronic device (20').

14. The system (100) according to claim 13, wherein:
- the exercise machine (10') comprises a respective first data processing block (11') and a respective first data communication block (12') operatively connected to the first data processing block (11'), the first data processing block (11') being configured to communicate data by means of said first data communication block (12');
- the at least one user's portable electronic device (20') comprises a respective second data processing block (21') and a respective second data communication block (22') operatively connected to the second data processing block (21'), said second data processing block (21') being configured to communicate data by means of said second data communication block (22').

15. The system (100) according to claim 14, wherein the at least one data processing unit (2) corresponds to one of the first data processing block (11') of the exercise machine (10') and the second data processing block (21') of the at least one portable electronic device (20') or is distinct from both the first data processing block (11') of the exercise machine (10') and the second data processing block (21') of the at least one portable electronic device (20'),
if the at least one data processing unit (2) corresponds to one of the first data processing block (11') of the exercise machine (10') and the second data processing block (21') of the at least one portable electronic device (20'), the operations included in the operation of the at least one first data processing unit (2) are delegated to the data processing block to which the at least one first data processing unit (2) corresponds, in this case the at least one first data processing unit (2) being configured to determine the value (Q1) of the amount of physical exercise as a function of data representative of the physical activity performed by the user tracked by the first electronic assembly (1) and additional data representative of the physical activity performed by the user tracked by the second electronic assembly (1'),
if the at least one first data processing unit (2) is distinct from both the first data processing block (11') of the exercise machine (10') and the second data processing block (21') of the at least one portable electronic device (20'), the at least one first data processing unit (2) is configured to determine the value (Q1) of the amount of physical exercise as a function of a first value (Q1') of the amount of physical exercise determined by the first data processing block (11) as a function of the data representative of the physical activity performed by the user tracked by the first electronic assembly (1) and a second value (Q1") of the amount of physical exercise determined by the second data processing block (21') as a function of the additional data representative of the physical activity performed by the user tracked by the second electronic assembly (1'), the operation of determining the value (Q1) of the amount of physical exercise performed by the user is partially delegated to the data processing blocks (11, 21') while all other operations, included in the operation of the at least one first data processing unit (2), remain the responsibility of the at least one first data processing unit (2),
alternatively, the at least one first data processing unit (2) is configured to determine the value (Q1) of the amount of physical exercise as a function of the data representative of the physical activity performed by the user tracked by the first electronic assembly (1) and the additional data representative of the physical activity performed by the user tracked by the second electronic assembly (1').

16. The system (100) according to claim 15, wherein the exercise machine (10') comprises a respective first user interface module (13') operatively connected to the first data processing block (11') of the exercise machine (10') and the at least one user's portable electronic device (20') comprises a respective second user interface module (23') operatively connected to the second data processing block (21'),
the at least one first data processing unit (2) being configured to send, based on the comparison of the determined value (Q1) of the amount of physical exercise with the set reference value (R1), a first notification message (N1) comprising the first information (AP-1) or the second information (AP-2),
the first data processing block (11') of the exercise machine (10') being configured to provide the user with the first notification message (N1) by means of the first user interface module (13') of the exercise machine (10'),
the second data processing block (21') of the at least one portable electronic device (20') being configured to provide the user with the first notification message (N1) by means of the second user interface module (23').

17. The system (100) according to any one of the preceding claims, wherein, when providing the user and/or personnel responsible for issuing an authorization to purchase the drug with the generated first information (AP-1) representative of the user's authorization to take the next drug dose or when generating and providing the user and/or personnel responsible for issuing an authorization to take the drug with the second information (AP-2) representative of a non-authorization for the user to take the next drug dose, the at least one first data processing unit (2) is configured to store the generated first information (AP-1), representative of the authorization for the user to take the next drug dose, or the generated second information (AP-2), representative of the non-authorization for the user to take the next drug dose, in the at least one first memory unit (3) in a first reserved area (A1) for consultation by the user and/or personnel responsible for issuing an authorization to purchase the drug.

18. A method (600) for managing the authorization to proceed with an intake of a drug dose by a user as a function of the physical activity performed by the user, said method (600) comprising steps of:
- comparing (601), by at least one first data processing unit (2) operatively connected to a first electronic assembly (1) for tracking the physical activity performed by the user, a value (Q1) of the amount of physical exercise performed by the user, starting from a first time instant in which a drug dose was taken by the user, with a set reference value (R1), the value (Q1) of the amount of physical exercise performed by the user having been determined, starting from the first time instant in which a drug dose was taken by the user, as a function of data representative of the physical activity performed by the user tracked by said first electronic assembly (1) for tracking the physical activity performed by the user;
- storing (602), by the at least one first data processing unit (2), in at least one first memory unit (3) operatively connected to the at least one first data processing unit (2), the determined value (Q1) of the amount of physical exercise performed by the user;
(Y) if the determined value (Q1) of the amount of physical exercise is greater than or equal to the set reference value (R1):
- generating (603) by said at least one first data processing unit (2), a first information (AP-1) representative of an authorization for the user to take the next drug dose;
- providing (604) by said at least one first data processing unit (2), the user and/or personnel responsible for issuing an authorization to purchase the drug with the generated first information (AP-1), representative of the authorization to proceed with the intake by the user of the next drug dose;
(N) if the determined value (Q1) of the amount of physical exercise is less than the set reference value (R1):
- generating and providing (605) by said at least one first data processing unit (2), the user and/or personnel responsible for issuing an authorization to purchase the drug with a second information (AP-2) representative of a non-authorization for the user to take the next drug dose or not generating a first information (AP-1) representative of the authorization for the user to take the next drug dose.
